# EUROPEAN PATENT APPLICATION

(11) **EP 4 606 305 A1**
(43) Date of publication of application: **27.08.2025**
(21) Application number: 25158506.3
(22) Date of filing: 18.02.2025
(51) Int. Cl.: A61B 5/363, A61B 5/366, A61B 5/00, A61B 5/35, G16H 50/70

(54) **PACED VENTRICULAR TACHYCARDIA DETECTION**

(30) Priority: 26.02.2024 US 202463557926 P
(71) Applicant: Drägerwerk AG & Co. KGaA, 23558 Lübeck (DE)
(72) Inventor: YU, Chen, Andover, 01810 (US); WEIDNER, Devin, Andover, 01810 (US); BEAN, Kristen, Andover, 01810 (US); LU, Haisheng, Andover, 01810 (US)
(74) Representative: Haseltine Lake Kempner LLP

(57) **Abstract**

A physiological monitoring system collects electrocardiogram ("ECG") data from through monitoring sensors via a sensor interface. A machine learning model identifies characteristics of the ECG data that may be attributable to a cardiovascular device used by the patient. At least one normal ECG template for the individual patient is created and continuously updated by the machine learning model. Abnormal ECG data sets are analyzed using knowledge-based beat classifiers to identify specific arrhythmias. A data structure stores responses to the specific arrhythmias based on their likely effects on the patient. For example, the response to a dangerous arrhythmia may be an emergency alarm, while the response to a temporary arrhythmia quickly corrected by the patient's pacemaker may be a non-urgent message or a note to file.

## Description

### TECHNICAL FIELD

The present disclosure relates generally to the field of medical monitoring of physiological parameters in a patient. More particularly, it relates to detecting, analyzing, and responding to abnormalities in the electrocardiogram of a patient who uses a cardiovascular device.

### BACKGROUND

This section introduces information about art that may be related to the subject matter being described and/or claimed. It is intended to provide context to aid understanding of the disclosure. This potentially related "background art" may or may not qualify as "prior art" under the relevant patent law. Thus, its discussion in this section is not an admission of prior art and should not be construed as such.

Many advances in medical diagnostics and treatment have resulted from clinicians' ability to access and interpret measurements of observable phenomena correlated to various aspects of a patient's state of health (collectively, "physiological parameters"). Collecting and comparing measurements taken at different times or under different conditions ("monitoring" the physiological parameters) may reveal meaningful trends, sensitivities, or possible prognoses that enable better-informed ongoing care of the patient.

Automation of the measurement and monitoring of physiological parameters confers some valuable benefits. For example, subjectivity-related variations are reduced because measuring devices can be calibrated to uniform standards. Artificial sensors may be able to measure physiological parameters with greater sensitivity over a wider range than human sight, hearing, or touch. Delegating the monitoring of a physiological parameter to a device frees the clinician to observe some other parameter of the patient, or to monitor several patients simultaneously. Other benefits may also result from automation of medical monitoring besides those mentioned here. Therefore, the development of medical monitoring devices, systems, and processes continues to grow and evolve with the needs of patients and the availability of new technologies.

The human heartbeat is one of the most fundamental vital signs, and heart disease is one of the most common causes of death worldwide. A patient whose heart stops may die unless it can be restarted in a sufficiently short time. Many types of heart disease, however, may be detected by known early-warning signs well before they progress enough to stop the heart. Various steps may then be taken to mitigate the danger. Continued monitoring allows the mitigation measures to be evaluated for effectiveness.

One type of early warning-signs is arrhythmia, where the heart beats at an abnormal rate (such as tachycardia, which is too fast, or bradycardia, which is too slow) or with an abnormal rhythm (skipped beats or extra beats within the repeated cycle). There are many different types and levels of arrhythmia, from occasional mild anomalies in otherwise healthy hearts to serious conditions needing immediate medical attention. They may be caused by other heart problems, other diseases, medications, aging, or other factors. Their effects felt by the patient range from slight discomfort to incapacitation. Therefore, correctly identifying the type of arrythmia experienced by the patient may be crucial to diagnosing the underlying problem and implementing effective countermeasures.

An electrocardiogram ("ECG") is one of the most common measurements for detecting and analyzing arrhythmia (although it has numerous other applications). When the heart beats, heart muscles around each of the four heart chambers (right and left atria and right and left ventricles) may contract and relax in sequence. During the contraction that forces blood out of each chamber, the cells of the involved muscle tissue may electrically depolarize, or discharge. The resulting electric field pulses propagate outward from the heart to produce small but detectable changes in voltage, on the order of microvolts, at the surface of the skin. ECG measures these voltage changes through one or more monitoring sensors placed in contact with the skin. The voltage may be measured over sufficient time to collect a data set spanning several heartbeat cycles (for example, 2.5 to 10 seconds). The collected data set of voltage vs. time may be called an "ECG" or a "trace" or "waveform." The term "ECG" may also refer to the instrument that collects the data, or to the process of collecting the data.

Historically, ECGs were plotted on graph paper with a pen plotter and read by eye. The plotters were often calibrated to a standard speed and scale so that ECGs collected under different conditions could be easily compared. Reading and interpreting the ECG was a skill that clinicians were required to learn. Beyond the basics of identifying "average normal" ECGs and some common abnormalities, human readers varied widely in the amount of information and level of detail they could extract from ECGs and the accuracy of the inferences about a patient's health they could extrapolate. Besides the extremely wide variety of abnormalities that could occur, some individual patients had atypical ECGs as their "personal normal" without underlying disease. Not all of the best ECG readers, informed by experience or a "good eye," could effectively teach others to replicate their success.

The more complicated the trace, the more difficult it may be to interpret. Additional complexity may be added to an ECG trace if the action of a cardiovascular device affects the voltage variations being measured. For example, pacemakers may provide timed electrical pulses to maintain a steady rhythm in a heart that might otherwise develop arrhythmia. Some pacemakers operate continuously, while others may allow a patient's heart to self-regulate until its built-in sensor detects an abnormality such as tachycardia (beating too quickly) or bradycardia (beating too slowly). Like the "intrinsic" electrical signals directly generated by the heart muscles, the pacing pulses are detectable by ECG at the surface of the skin, and the resulting trace reflects a mixed, or combined, signal from both the heart and the pacemaker. This mixed data set may be more complex than one produced by the patient's heart alone. Other types of cardiovascular devices add their own complexities. Pacing pulses and other contributions of cardiovascular devices may be generally termed "stimulation artifacts" ("SAs"). Unless SAs can be detected and analyzed in the ECG of a device-using patient, an ECG trace of a heart rhythm being successfully corrected by a device may be mistaken for an abnormal trace, causing unnecessary treatments to be recommended or administered.

Although cardiovascular devices are intended to regulate the heart rhythm, some may permit, or even cause, arrhythmia (for example, in pacemaker-mediated tachycardia or "PMT"). Some of these scenarios result from device failures or malfunctions needing correction. However, some newer devices may be designed to temporarily permit one type of arrhythmia, such as tachycardia, in order to prevent a more dangerous one such as fibrillation. As newer cardiovascular devices operate on increasingly sophisticated algorithms, accurate analysis of the resulting complex ECG traces would allow clinicians to discover whether a patient's device is functioning as intended.

Computers analyzing digitized ECG traces have the advantage of being able to mathematically separate mixed signals such as that of a patient's heart and a cardiovascular device. They can also process the data to enhance details, transform it from the time domain to the frequency domain, interpolate, extrapolate, curve-fit, compare different data sets, and use many other techniques to extract information from the collected signal. Moreover, computers can perform many analyses at sufficient speed to deliver the results to clinicians in real time, enabling the clinicians to timely intervene i. However, the variety of natural and device-modulated ECG traces that may be produced by the general patient population is still too vast to be satisfactorily handled by conventional human-written computer instructions based on declarative or procedural knowledge. An alternative that reliably alerts clinicians when a patient's life is in immediate danger, while minimizing the occurrence of false alarms that may waste resources and cause unnecessary pain to patients, would be a valuable addition to patient-monitoring technology.

### SUMMARY

The following simplified summary is intended to provide a basic understanding of some aspects of the disclosure that may be included in the appended claims. It is not meant as an exhaustive overview of the subject matter, a complete list of key or critical elements, or a recitation of the limits of what may be claimed. Its only purpose is to introduce some of the concepts and terminology to be discussed in more depth in the Detailed Description.

A physiological monitoring system collects electrocardiogram ("ECG") data from through monitoring sensors via a sensor interface. Analysis software identifies characteristics of the ECG data that may be attributable to a cardiovascular device used by the patient. A personalized template of a normal ECG range for the patient and device is created and continuously updated by machine learning.

Abnormal ECG data sets (those that do not match the patient's normal template, and optionally those collected before the patient's normal template is learned) are analyzed by one or more stored beat classifiers representing particular arrhythmias. The comparison determines what effect the arrhythmia is likely to have on the patient and prompts the system to respond accordingly. For example, arrhythmias that cause severe distress or risk may prompt emergency alarms to clinicians; arrhythmias that cause moderate discomfort may prompt real-time non-emergency alerts to clinicians; and arrhythmias that are quickly and painlessly corrected by the cardiovascular device may prompt a non-urgent message.

A disclosed method of detecting arrhythmia in electrocardiogram ("ECG") data includes at least (1) collecting at least one of (a) an intrinsic ECG signal from a patient and (b) a stimulation artifact from a cardiovascular device used by the patient; (2) creating a normal template from successive sets of the ECG data by unsupervised machine learning; (3) detecting whether a specific arrhythmia is present in the ECG data by applying at least one knowledge-based beat classifier; and (4) executing a response corresponding to the expected effect of the specific arrhythmia on the patient.

A disclosed physiological monitoring system includes at least (1) at least one electrocardiogram ("ECG") port; (2) a processor communicatively coupled to the ECG port; and (3) a memory communicatively coupled to the ECG port and encoded with (a) a data structure including (i) a beat classifier to detect a specific arrhythmia in ECG data; (ii) a corresponding response linked to the predefined arrythmia; (iii) a normal template learned from ECG data; and (iv) a set of instructions that, when executed by the processor, cause the processor to (A) collect an ECG data set, including at least one of (I) an intrinsic ECG signal from a patient and (II) a stimulation artifact from a cardiovascular device of the patient, through the ECG port; (B) compare the ECG data set to the normal template; and (C) if the ECG data set matches the normal template, update the normal template with the ECG data set.

A disclosed non-transitory computer-readable storage medium contains instructions that, when executed, cause a processor to at least (1) collect electrocardiogram ("ECG") data including at least one of an intrinsic ECG signal from a patient and a stimulation artifact from a cardiovascular device used by the patient; (2) create a normal template from successive sets of the ECG data by unsupervised machine learning; (3) detect whether a specific arrhythmia is present in the ECG data by applying at least one knowledge-based beat classifier; and (4) execute a response corresponding to the expected effect of the specific arrhythmia on the patient.

Additional methods, systems, and non-transitory storage media are described in this document and the accompanying drawings.

The above is intended as an introduction to some possible implementations of the invented subject matter. It does not limit the scope of protection afforded by the claims.

### BRIEF DESCRIPTION OF THE DRAWINGS

The drawings illustrate specific implementations described in detail by way of example. Neither the drawings nor the corresponding descriptions are intended to limit the scope of protected subject matter. On the contrary, the intent is to protect all modifications, equivalents, and alternatives falling within the spirit and scope of the appended claims.
FIG. 1 schematically illustrates a physiological monitoring system according to some embodiments.
FIG. 2 schematically illustrates a patient undergoing ECG monitoring while using a cardiovascular device.
FIG. 3 presents examples of selected kinds of ECG traces and their structural features that may be analyzed by some disclosed methods.
FIG. 4 is a general overview of the analysis model according to some embodiments.
FIG. 5 is a conceptual diagram of a memory configured for carrying out some embodiments of the disclosed methods.
FIG. 6 is a process chart of instructions for a normal-template learning session according to some disclosed methods.
FIG. 7 is a decision tree of an ECG diagnostic analysis using knowledge-based beat classifiers according to some disclosed methods.
FIG. 8 is an example ECG trace of intrinsic tachycardia successfully corrected by a cardiovascular device.
FIG. 9 is an example ECG trace of intrinsic tachycardia that a cardiovascular device unsuccessfully attempts to correct.
FIG. 10 is an example ECG trace of endless-loop tachycardia ("ELT"), a type of pacemaker-mediated tachycardia.
FIG. 11 is an example ECG trace of pacemaker-mediated exit-block ventricular tachycardia.

In the drawings, like reference numbers generally indicate elements that are functionally similar, structurally similar, or both.

### DETAILED DESCRIPTION

Unless otherwise defined, all terms including technical and/or scientific terms used herein have the same meaning as commonly understood by one of ordinary skill in the art of the present disclosure. Definitions of some art-specific terms as used in this document are presented below. Definitions of more general or art-agnostic patent terms are presented near the end of the document,

"Abnormal" may describe an ECG whose characteristics depart beyond an allowable margin, either from the patient's stored personalized normal ECG template or from one or more other stored references or standards.

"Allowable margin" may refer to a maximum error or departure that may be measured between one data set and another, or between one data set and an average of multiple others, while still considering the data sets to match. This maximum may be preset, derived from a machine learning process, adjusted by a clinician, or have some other origin.

"Collecting ECG data," as used herein, refers to, without limitation, manipulating an analog signal to meet the requirements of a next stage for further processing. ECG signal processing may include converting between analog and digital realms (e.g., via an analog-to-digital or digital-to-analog converter), amplification, filtering, converting, biasing, range matching, isolation and any other processes required to make a sensor output suitable for processing.

"Computer" as used herein may be any known computing apparatus or device, integrated or distributed, physical or virtual, digital or analog, using binary, ternary, decimal, hexadecimal, or any suitable notation. It may include a processor, a way to enter input, a way to access output, and optionally a way to store and/or retrieve data.

"Device-modulated" refers to a physiological measurement data set that includes characteristics attributable to a medical device in use by the patient. For example, an ECG of a patient using a pacemaker may exhibit regularly spaced sharp spikes recognizable as "pacing pulses" generated by the pacemaker.

"ECG," if used as a noun, may refer to a machine that makes ECG measurements, or to its output. If used as an adjective, it may refer to anything related to the process of ECG measurement.

"Fused beats" in their broadest sense are ECG features that appear when the myocardium is triggered by more than one source. For purposes of this disclosure, however, "fused beat" refers to a stimulation artifact, such as a pacemaker pulse, coinciding so closely with the QRS complex that the intrinsic and stimulated contributions are difficult to resolve. For example, instead of having a discernible extra peak, the QRS complex may only look broadened.

"Link," in a programming context, may mean any association or relationship between two or more pieces of information that can be sensed and acted upon by a running program. The link may reside in a non-transient data structure or in a set of processing instructions. The link need not be a distinct item of code but may be a proximity or relative position of the two pieces of information, as in a table or register. By contrast, in the context of communications hardware, a "link" may be any suitable channel over which the communicating components may send or receive data.

"Machine" herein may be any device, physical or virtual, that transmits or modifies any type of energy to perform or assist in the performance of human tasks.

"Machine learning" may refer to algorithms that find patterns in data (often large amounts of data). Once the patterns are found, the algorithms can fit newly input data into the pattern or predict how various changes might affect the pattern. Instead of being explicitly programmed to fit functions in a predefined way, machine learning algorithms can use statistical methods to adapt their approaches based on "experience" (previous results). Machine learning models can solve problems that would be too costly, difficult, or time-consuming for traditional algorithms devised by human programmers, such as facial recognition, speech recognition, and customer preference prediction. (Although a subset of machine learning known as "deep learning" may make use of artificial neural networks (ANNs), a wide range of machine learning models succeed without ANNs).

A "match" between data sets, as used herein, shall not necessarily mean an exact match, but allows for a tolerable margin of error that may be predefined and stored in a data structure or derivable by the processor according to stored instructions being executed.

A "model" in machine learning is a program that has learned a task, such as recognizing certain patterns, by being trained on one or more training sets.

A "module" may include hardware, software, or a combination of both assembled to implement a desired function. A degree of interchangeability between software and hardware is well known in the art and finds applicability in technological contexts such as those disclosed herein. Thus, a module may be implemented in, for example, electronic logic circuits comprised of electronic components such as transistors, resistors, inductors, capacitors, etc. Alternatively, a module may be implemented in software in the form of executable instructions stored by a processor-based resource. Alternatively, a module may be implemented in an integrated circuit such as an ASIC, an EPROM, or an EEPROM. Alternatively, a module may combine some or all of these technologies.

"Pacing-dependent" may describe a patient whose pacemaker or other cardiovascular device is set to operate at all times because their heart is unlikely to self-regulate in the absence of such assistance.

A "sensor" may be a transducer which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

A "training set" may be a data set input to a machine learning program to enable it to learn a new task. The data may be labeled (inputs linked to predefined results), unlabeled (input values only), or a mixture of the two. Once the program has adjusted its rules and data structures to successfully perform the task (evolved into a "model") it is given a "test set," different from the training set, to similarly analyze. In an ongoing process that builds the model's experience, part or all of a test set may become a training set for a subsequent test set.

Illustrative examples of the subject matter claimed below are disclosed. In the interest of clarity, not all features of an actual implementation are described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

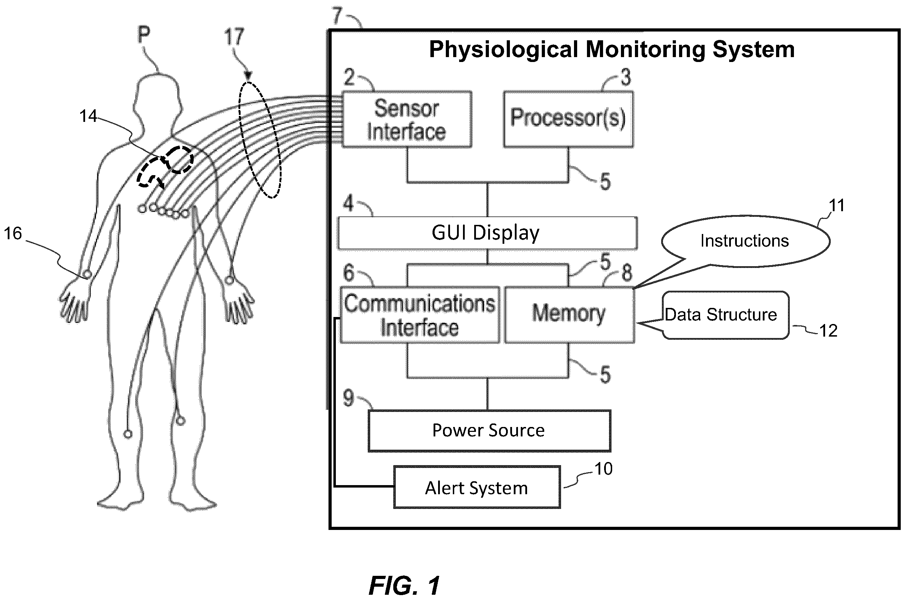

FIG. 1 schematically illustrates a physiological monitoring system according to some embodiments. First an overview of the system will be presented, after which each component and its options will be presented in more detail.

Physiological monitoring system 7 receives monitoring signals at sensor interface 2 from sensors 16 over leads 17. Sensor interface 2 may include one or more ECG ports for receiving ECG signals. Sensors 16 are strategically positioned relative to patient P to monitor patient P's physiological parameters. One or more of sensors 16 may be ECG sensors. Patient P uses cardiovascular device 14 which, when active, may affect ECG signals sensed by sensors 16. As a non-limiting example, cardiovascular device 14 may include a pacemaker or implanted cardioverter defibrillator ("ICD").

Physiological monitoring system 7 may include modules 2-6 and 8-10 interconnected by internal buses 5 to perform various functions. The modules may include hardware, software, or a combination. Internal buses 5 may carry data signals, control signals, power, or combinations thereof between the various hardware modules. Sensor interface 2 receives physiological monitoring signals, which may include ECG monitoring signals, through leads 17. One or more processors 3 may organize and analyze physiological monitoring data received at sensor interface 2 and may also control the general operations of physiological monitoring system 7. A graphic user interface ("GUI") display 4 may be configured to display various patient data, sensor data, and hospital or patient care information. Moreover, the user may query, control, and otherwise interact with physiological monitoring system 7 through the GUI function of GUI display 4. Power source 9 supplies power to at least some of the power-consuming components inside or connected to physiological monitoring system 7.

A communications interface 6 may permit physiological monitoring system 7 to communicate with one or more external devices or networks (not shown) directly or indirectly. For example, communications interface 6 may allow information from GUI display 4 to be viewed on a remote monitor (not shown). Additionally, or alternatively, communications interface 6 may send alert messages or other signals from alert system 10 to devices or destinations attended by clinicians.

Memory 8 may receive data from processor(s) 3 or other sources, store it for a short or long term in one or more structures, and allow processor(s) 3 or other destinations to access the data. As a non-limiting example, a data structure 12 in memory 8 may include a set of stored beat classifiers, each associated with a corresponding response by a link. Some of the responses may include actions to be performed by alert system 10. As another non-limiting example, memory 8 may store instructions 11 for processor(s) 3 to execute, such as organizing ECG data into ECG data sets, identifying the contributions of cardiovascular devices 14, accessing data structure 12 to check for indicators of arrhythmia, activating alert system 10 if a selected response 13 includes it, and applying a machine learning model to update a personal normal ECG template for patient P.

Each of the illustrated elements may be embodied in various suitable ways whole remaining within the scope of the appended claims, as follows:

Conductive leads 17 coupling sensors 16 to sensor interface 2 may include any type of suitable signal-carrying cable (e.g., single wire, twisted pair, coaxial, fiber-optic). Additionally, or alternatively, one or more sensors 16 may be wirelessly connected to sensor interface 2, in which case sensor interface 2 may include circuity for receiving and/or transmitting data using any suitable wireless protocol such as Wi-Fi^{®} connection, cellular data, or a near-field communication protocol such as Bluetooth^{®}.

The data signals received from sensors 16 may be analog signals. For example, the data signals for the ECG may be input to sensor interface 2, which can include an ECG data acquisition circuit (not shown separately in FIG. 1). An ECG data acquisition circuit may include amplifying and filtering circuity as well as analog-to-digital ("A/D") circuity that converts the analog signal to a digital signal using amplification, filtering, and A/D conversion methods. In the event that ECG sensor 16 is a wireless sensor, sensor interface 2 may receive the data signals from a wireless communication module (not shown in FIG. 1). Thus, sensor interface 2 may be configured to interface with one or more sensors 16 and receive sensor data therefrom.

As alluded to above, successive ECG data sets are acquired by receiving an ECG signal from one of the ECG sensors 16 through its lead 17, then processing the acquired ECG signal using processor(s) 3 of physiological monitoring device 7. The acquired ECG signal may be buffered in as data in memory 8 or processed in real time. Similarly, the ECG data sets, once processed, may be buffered as data in memory 8 as needed or desired.

Accordingly, the ECG signal and the ECG data sets may be processed, displayed, or otherwise handled or manipulated in real time or near real time. The term "near real time" means as closed to real time as the computing resources of a given embodiment permit. Some embodiments may also store the ECG signal, impedance respiration signal, or other data in the memory 8 for a longer term. For example, some embodiments may manage processing resources by storing data before pushing it out. Similarly, some embodiments may store data pulled from other locations.

Processor(s) 3 may be any suitable processor-based resource. They may be, but are not limited to, a central processing unit ("CPU"), a hardware microprocessor, a multi-core processor, a single core processor, a field programmable gate array ("FPGA"), a controller, a microcontroller, an application specific integrated circuit ("ASIC"), a digital signal processor ("DSP"), or other similar processing device capable of executing any type of instructions, algorithms, or software for controlling the operation and performing the functions of physiological monitoring system 7. In some embodiments, processor(s) 3 may comprise a processor chipset including, for example and without limitation, one or more co-processors. The processor may be centralized or distributed among two or more chips, boards, or other locations.

GUI display 4 may interface with one or more input devices (not shown) such as a keyboard, pointing or tracking device, microphone, or camera. The display may include a liquid crystal display ("LCD"), cathode ray tube ("CRT") display, thin film transistor ("TFT") display, light-emitting diode ("LED") display, high definition ("HD") display, or other similar display device that may include touch screen capabilities. GUI display 4 may provide a means for inputting instructions or information directly to physiological monitoring system 7. The patient information displayed may, for example, relate to the measured physiological parameters of patient P (e.g., ECG readings).

Communications interface 6 may permit physiological monitoring system 7 to directly or indirectly communicate with one or more computing networks and devices, workstations, consoles, computers, monitoring equipment, alert systems, and/or mobile devices (e.g., a mobile phone, tablet, or other hand-held display device). Communications interface 6 may include various network cards, interfaces, communication channels, cloud, antennas, and/or circuitry to permit wired and wireless communications with such computing networks and devices. Communications interface 6 may be used to implement, for example, a Bluetooth^{®} connection, a cellular network connection, and/or a Wi-Fi^{®} connection with such computing networks and devices. Example wireless communication connections implemented using communication interface 6 include wireless connections that operate in accordance with, but are not limited to, IEEE 802.11 protocol, Radio Frequency For Consumer Electronics ("RF4CE") protocol, and/or IEEE 802.15.4 protocol (e.g., Zigbee^{®} protocol). In essence, any wireless communication protocol may be used.

Additionally, communications interface 6 may permit direct (i.e., device-to-device) communications (e.g., messaging, signal exchange, etc.) to and from physiological monitoring system 7 using, for example, a universal serial bus ("USB") connection or other communication protocol interface. Communication interface 6 may also permit direct device-to-device connection to other devices such as to a tablet, computer, or similar electronic device; or to an external storage device or memory.

Memory 8 may be a single memory device or a group of co-located or distributed memory devices. The locations may include, without limitation, on-chip with the processor or any of the other functional modules of physiological monitoring system 7, or off-chip in one or more dedicated memory modules. Memory 8 may be removable or installed or a combination thereof, and may be volatile or non-volatile or a combination thereof. Memory 8 may be, for example, a RAM, a memory buffer, a hard drive, a database, an EPROM, an EEPROM, a ROM, a flash memory, a hard disk, or any other non-transitory computer readable medium capable of storing data and instructions.

Power source 9 may include a self-contained power source such as a battery pack and/or include an interface to be powered through an electrical outlet, either directly or by way of a monitor mount or other power connection system. Power source 9 may also be a rechargeable battery that can be detached allowing for replacement. A back-up power source may be connected, such as an uninterruptable power supply, or built-in, such as a back-up battery or supercapacitor, to provide continuous power to physiological monitoring system 7 during power failure, battery replacement, or other power loss.

Physiological monitoring system 7 may optionally be deployed as part of a larger health-facility management system with functions that may include, but are not necessarily limited to, physiological monitoring. The health-facility management system may include a group of physical virtual server and client devices connected in a network or cloud. Physiological monitoring system 7 may exchange information with health-facility management system over any suitable wired or wireless communication link.

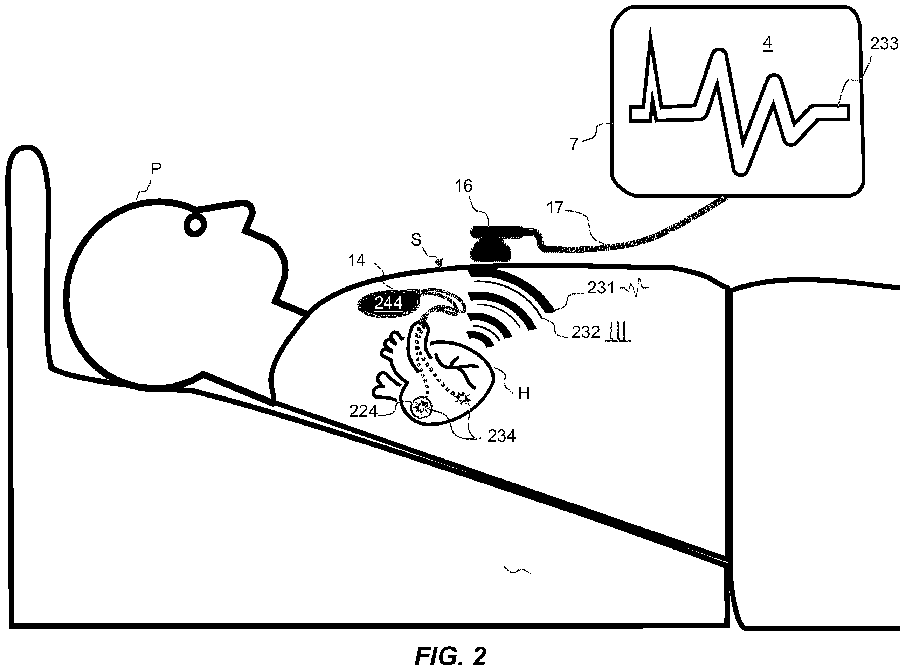

FIG. 2 schematically illustrates a patient undergoing ECG monitoring while using a cardiovascular device. Patient P's heart H may produce intrinsic ECG signal 231 when the muscles around the chambers of heart H electrically depolarize, which triggers a mechanical contraction that pushes blood in and out of the chambers, The resulting electrical signal 231 may propagate outward from heart H until it reaches skin surface S, where it may be detected by a sensor such as monitoring sensor 16. Monitoring sensor 16 may contact skin surface S through a layer of conductive gel (not shown) to improve signal reception.

Meanwhile, patient P's cardiovascular device 14 may include pulse generator module 244, pacing leads and electrode(s) 234, and some type of sensing capability 224. Pulse generator module 244 houses the pulse generating hardware, a storage medium with control software and firmware, and a power source (usually a battery). When sensing capability 224 senses an arrhythmia, or on other occasions such as when the device's programming prompts it to pace or otherwise stimulate the myocardium of heart H, pulse generator 244 sends a corrective electrical signal 232 to heart H through pacing leads and electrodes 234.

Although conceptual and not to scale, cardiovascular device 14 as shown here schematically represents an implanted transvenous pacemaker, ICD, or the like. Cardiovascular device 14 may be entirely implanted in the body, with leads and electrodes 234 threaded through a central vein into the interior of heart H. There, electrodes 234 stimulate, and sensing mechanism 234 senses, electrical activity in the inner wall of one or more heart chambers directly.

Cardiovascular devices may be configured in a variety of different ways to address the needs of different patients. Some are intended to be temporarily installed in an emergency, while others are long-term or permanent. In some cardiovascular devices 14, a single pair of electrical contacts performs both the sensing and the pacing functions. In others the functions may be performed by different subassemblies. Pacing may be unipolar or bipolar. The pacing pulses may be delivered to a single chamber of heart H (usually the right ventricle) or two chambers (either the right atrium and right ventricle in dual-chamber devices, or the right and left ventricle in biventricular devices). Dual-chamber devices may operate in various modes, such as AS-VP (atrial-sensed, ventricular-paced), AP-VS (atrial-paced, ventricular-sensed), AP-VP (atrial-paced, ventricular-paced), and fused-beat (ventricular-paced with different timing than the other VP cases).

Biventricular cardiovascular devices may be used in cardiac resynchronization therapy ("CRT"). In one example of a CRT device, a left ventricular ("LV") pacing lead may be added to a standard pacemaker or ICD that already has a right ventricular ("RV") pacing lead and optionally a right atrial lead. Unlike some other cardiovascular devices that only activate when a need is detected, CRT is often most effective in an "always on" mode: that is, when the ventricle is paced as close to 100% of the time as possible.

Leadless cardiovascular devices are integrated assemblies entirely inserted into a chamber of heart H, often through a vein. Subcutaneous cardiovascular devices may be implanted under the skin (for example, below the armpit on the side of the chest), with an electrode running along the sternum rather than impinging on the heart itself.

For any of these configurations, when the corrective signal propagates from electrodes 234 through the muscles of heart H, it continues to propagate to external skin surface S as device signal 232. Thus, when cardiovascular device 14 is active, ECG monitoring sensor 16 receives stimulation artifact 232 concurrently with intrinsic ECG signal 231.

ECG signal(s) 231 and 232 received by monitoring sensor 16 are transmitted through sensor leads 17 (which, as mentioned above, may be any suitable cable or a wireless connection) to physiological monitoring system 7 (specifically to an ECG port of its sensor interface, not shown in this figure). Their combination, device-modulated signal 233, may then be displayed on GUI display 4 and analyzed by one or more processors of the physiological monitoring system (not shown in this figure).

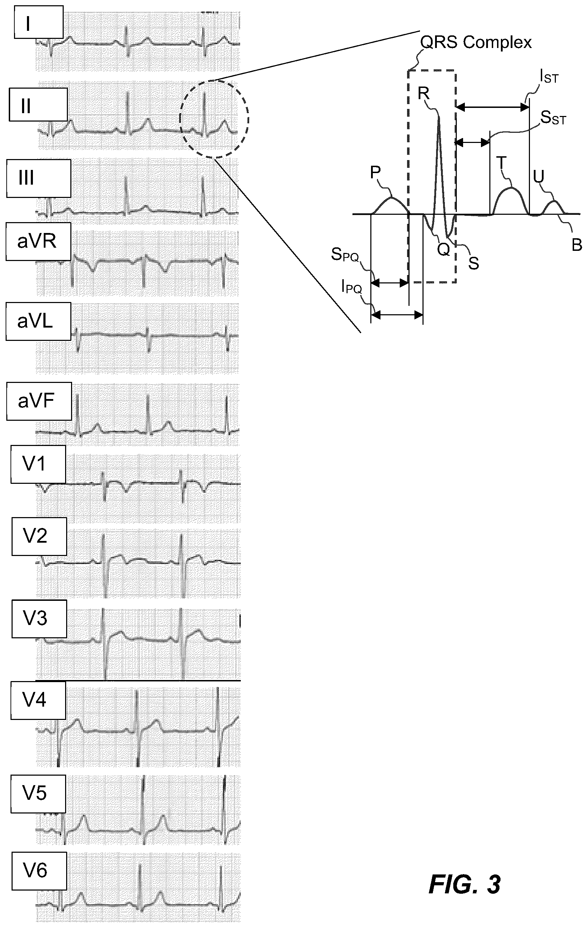

FIG. 3 presents examples of selected kinds of ECG traces and their structural features that may be analyzed by some disclosed methods. It introduces some intrinsic ECG features for the later discussion of recognizing arrhythmias and the patient-to-patient variations in normal ECG data sets. The graph series represents data sets from the channels of a 12-lead ECG, one of the diagnostic standards in cardiology. Voltage is plotted on the vertical axis, and time on the horizontal axis, of each graph in the series. The ECG is an indirect indicator of heart muscle activity because heart muscles contract in response to the electrical depolarization of their cells.

Data set I is a high lateral bipolar ECG measured between sensors on the patient's wrists (or shoulders). Data set II is a left inferior bipolar ECG measured between sensors on the patient's left wrist (or shoulder) and left ankle. Data set III is a right inferior bipolar ECG measured between sensors on the patient's right wrist (or shoulder) and left ankle (or lower abdomen).

Data sets aVR, aVL, and aVF may be derived from leads I, II, and III rather than being measured separately.

Data set V1 is a chest lead ECG measured by a sensor on the patient's right 4^{th} intercostal space (between the 4^{th} and 5^{th} right ribs). Data set V2 is a chest lead ECG measured by a sensor on the patient's left 4^{th} intercostal space. Data set V3 is a chest lead ECG measured by a sensor over the patient's left 5^{th} rib. Data set V4 is a chest lead ECG measured by a sensor on the patient's left 5^{th} intercostal space at the end nearest the sternum (breastbone). Data set V5 is a chest lead ECG measured by a sensor on the patient's left 5^{th} intercostal space at the end nearest the axilla (underarm). Data set V5 is a chest lead ECG measured by a sensor on the patient's left mid-axillar line (lateral centerline of the underarm).

All the data sets I-V6 were collected from the same patient at the same time. For a variety of reasons, they differ from each other. Different lead arrangements create additional differences. Therefore, analysis of each of the data sets may be informed by the context provided by the location of the originating leads.

The magnified view of the circled area of data set II shows a classical view of the characteristics, features, or "waves" (deviations from baseline B) that may be seen in an ECG of an example of a single heartbeat. Clinicians can extract information about a patient's heart health from the presence or absence, amplitude, width, shape, and spacing of the waves.

The small P wave is produced by electrical depolarization of the atria, originating in the sinoatrial ("SA") node. If one or both of the atria are paced by an "AP" mode of a cardiovascular device, a pacing pulse (narrow spike, not shown in this figure) may appear in or next to the P wave in the ECG.

During the baseline pause after the P wave, the atrial depolarization (ideally) spreads through the atrioventricular, or AV, node to the ventricles. The resulting electrical depolarization of the ventricles produces the "QRS complex," a wave series that may include a small downward Q wave, a tall upward R wave, and a small downward S wave. The beginning of the Q wave is known as "QRS onset." The QRS complex is one of the most informative parts of the ECG, containing clues to aid the diagnosis of various heart problems. Conventionally, even in ECGs where Q waves or S waves may be absent, whichever waves are present are nonetheless termed the "QRS complex." If one or both of the ventricles are paced by a "VP" mode of a cardiovascular device, a pacing pulse may appear at or near the QRS onset.

The small T wave that follows the QRS complex is produced by ventricular repolarization. The U wave after the T wave is generally much smaller than the T-wave, is often absent altogether. Its appearance sometimes indicates an underlying pathology and sometimes not. To complicate matters further, the T-wave in some patients may be split into two peaks and the second T-wave peak mistaken for a U-wave. The behavior of the U-wave when heart rate changes has been correlated with both tachycardia and bradycardia (fast and slow arrhythmias, respectively).

In a heart without arrhythmia, the QRS complex may be about 80-100ms long and, over the length of the ECG data set, the frequency of P waves and the frequency of QRS complexes are equal. Other commonly evaluated ECG dimensions include PQ segment S_{PQ}, PQ interval I_{PQ}, ST interval I_{ST}, and ST segment S_{ST}. Preferably, an algorithm that analyzes ECG data sets may detect and evaluate many of the characteristics examined by expert human readers, and perhaps extract some information not available to the human eye.

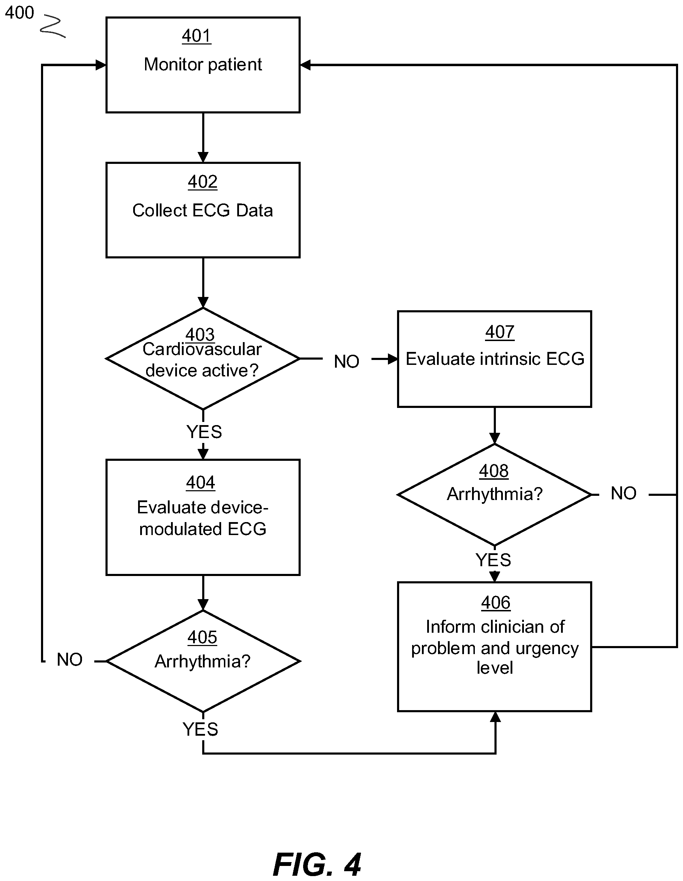

FIG. 4 is a general overview of the analysis model according to some embodiments. This introduction to the overall process 400 may provide a sense of orientation for the reader traversing the more detailed disclosures that will follow.

While the patient is being monitored in process 401, an ECG data set may be collected in process 402 and submitted to the analysis model as test data. The model determines whether the patient has a currently active cardiovascular device at branch point 403 by recognizing SAs and any other ECG characteristics attributable to such a device. In some embodiments this is done early in the analysis, or even as a first step before further classification, clustering, or other manipulation of the ECG data set.

If cardiovascular device activity is detected at branch point 403, the ECG data set is treated as device-modulated in process 404. In some embodiments, device-modulated data sets may be compared predominantly or exclusively to device-modulated templates based on device-modulated beat classifiers. If the device-modulated rhythm within acceptable margins is detected at branch point 405, the monitoring continues in a return to process 401. However, If a device-modulated tachycardia or other arrhythmia is detected at branch point 405, the system executes a stored response in process 406. The response is linked, via a data structure, to the detected arrhythmia and corresponds to its predicted effect on the patient. For example, an emergency alarm may be sent to a clinician if the patient may be in severe distress, or a non-emergency alert may be sent if the patient is likely to be uncomfortable but not in immediate danger.

If no cardiovascular device activity is detected at branch point 403, the ECG data set is treated as intrinsic (affected only by the actions of the patient's heart) in process 407. In some embodiments, intrinsic data sets may be compared predominantly or exclusively to intrinsic templates based on intrinsic beat classifiers. If the intrinsic rhythm within acceptable margins is detected at branch point 408, the monitoring continues in a return to process 401. However, If an intrinsic tachycardia or other arrhythmia is detected at branch point 408, the system executes a stored response in process 406. The response is linked, via a data structure, to the detected arrhythmia and corresponds to its predicted effect on the patient. For example, an emergency alarm may be sent to a clinician if the patient may be in severe distress, or a non-emergency alert may be sent if the patient is likely to be uncomfortable but not in immediate danger.

In some embodiments, each collected ECG data set may be converted to training data to help analyze subsequently collected test data sets and continuously build the expertise of the model. These options are described in more detail later in the disclosure.

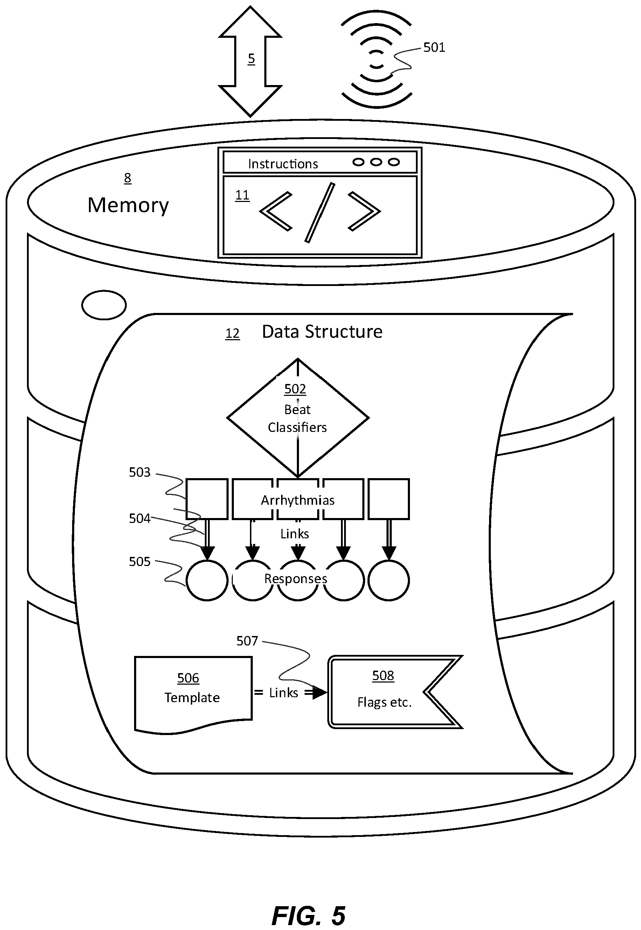

FIG. 5 is a conceptual diagram of a memory configured for carrying out some embodiments of the disclosed methods. Memory 8, previously introduced in FIG. 1, may be monolithic or distributed, integrated or detachable, part of a system-on-chip (SoC), realized as part of a virtual machine (VM), or any other suitable form of non-transitory machine-readable data storage. Memory 8 may communicate with one or more processors, controllers, transceivers, or other electronic components and devices through internal bus 5, wireless link 501, or any suitable type of permanent, detachable, or switchable communication link.

Memory 8 includes instructions 11 to be accessed and executed by one or more processors (not shown). Instructions 11 may include one or more machine learning models and one or more explicit programs. The machine learning models may use unsupervised learning, supervised learning, semi-supervised learning, or a combination.

Memory 8 also includes a data structure 12 that provides data to be accessed by one or more processors (not shown) during the execution of instructions 11. Knowledge-based beat classifiers 502 enable the model to recognize specific arrhythmias 503 in ECG data sets via explicit algorithms or supervised machine learning. Each specific arrhythmia 503 is associated via an information link 504 with a response 505. Each response 505 is based on the expected effect on the patient of specific arrhythmia 503 (e.g., emergency alarm for distress or danger, non-emergency alert for discomfort, log entry or non-urgent message if the condition self-corrects in a short time). Any suitable type of link, such as a lookup table or proximity, may be used.

Data structure 12 also includes templates 506, some of which may be created by unsupervised or semi-supervised machine learning. For example, ECGs collected for the same patient that exhibit acceptable rhythm (e.g., sinus beats or effective pacing by a pacemaker) may be used to create, and repeatedly update, the patient's normal template. Flags 508 identifying notable features of the ECGs, such as SAs and rhythms as well as an identifier of the patient, may be associated with templates 506 by any suitable type of link 507.

In some embodiments, data structure 12 has two separate substructures separating intrinsic from device-modulated data. That is, incoming intrinsic ECG data may be compared to intrinsic templates and analyzed with intrinsic beat classifiers, while incoming device-modulated ECG data may be compared to device-modulated templates and analyzed with device-modulated beat classifiers.

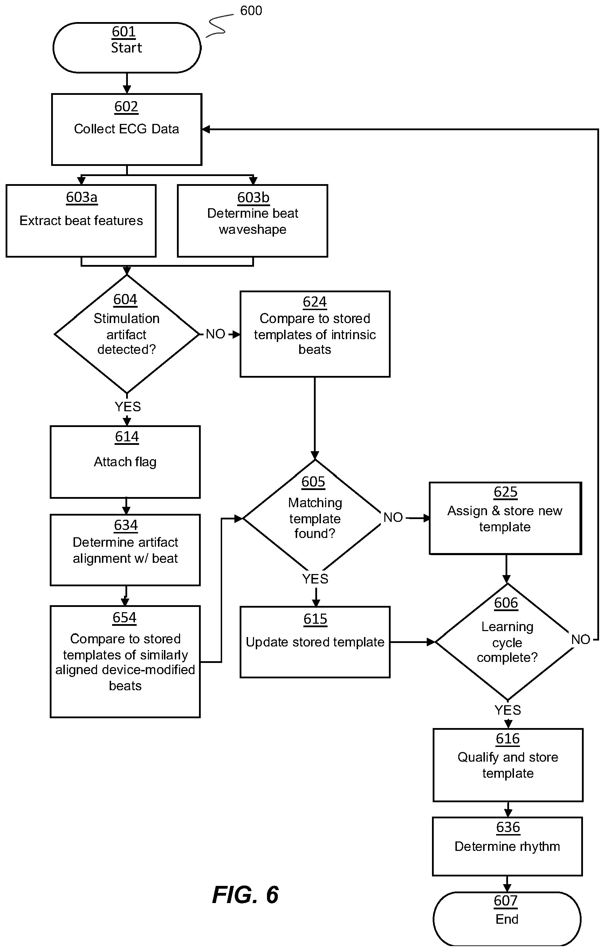

FIG. 6 is a process chart of instructions for a normal-template learning session according to some disclosed methods. Given the variations between patients and the different activities of cardiovascular devices, a patient's "normal" ECG may not be constant. The ECG may, for example, alter with a patient's physical or emotional state, or responsive to the effects of medication, or habituating itself to a cardiovascular device. To cope with this diversity, complexity, and changeability, machine learning may be harnessed to learn a personalized normal template that grows in range and flexibility as more ECG data is collected.

In some embodiments, the machine learning that creates and updates the personalized normal template may be unsupervised (trained from unlabeled data with only input values given, discovering hidden patterns and groupings) or semi-supervised (trained from a mixture of labeled and unlabeled data). The expected locations of SAs may be used in feature selection to reduce generalization error and stochastic noise.

At start-point 601 of process 600, the processor may collect an ECG data set from the patient in process 602. This test data set includes an intrinsic ECG signal generated by the patient's heart, and it may or may not also include one or more SAs contributed by a cardiovascular device used by the patient.

In processes 603a and 603b, the model extracts the beat features and waveshape from the ECG data set. The medical literature varies in what parameters it terms "features" compared to parts of the "waveshape," but the model may extract parameters including without limitation beat area, beat width, beat amplitude, QRS polarity, P-wave presence, P-wave time coupling with QRS complex, R-R interval, ST-elevation and depression, T-wave abnormalities, pathological Q-waves, R-R interval, S-T interval, P-R interval, and Q-T interval.

If the feature-and-waveshape analysis reveal no SAs attributable to cardiovascular devices at branch point 604, the results are compared to stored intrinsic templates in process 624. If, instead, one or more SAs reveal the activity of a cardiovascular device, a corresponding flag (e.g., "paced") may be attached to the data set in process 614. From there, the model analyzes the alignment of the SA with the intrinsic beat features (e.g., whether the SA comes before or after the QRS onset) at process 634. In process 654, the results are compared to any stored templates that are also flagged as device-modulated and have similar SA alignments.

Regardless of whether comparison process 624 or 654 is performed, if a matching stored template is found in the data structure at branch point 605, the new data is added to update the matching stored template in process 615. That is, the most recently analyzed test data becomes training data applied to analyze subsequently collected data. If no matching template is found, a new template is assigned and stored in process 625.

After updating the matching template in process 615 or assigning and storing a new template in process 625, the model determines at branch point 606 whether the current learning cycle is complete by applying a criterion, such as beat-to-beat correlation. For example, the algorithm may judge a learning cycle to be complete after 15 beats with correlation above a predetermined threshold value. If the learning cycle is still incomplete at branch point 606, the model returns to process 602 to collect more data. If it is complete, the model qualifies and stores the new or updated template(s) at process 616, determines the rhythm in process 636 before ending the learning session at end-point 607.

Each newly monitored patient may begin with a blank template, on which characteristics coalesce as successive ECG data sets are collected and analyzed. For example, the patient's normal ECG may exhibit one of the following common QRS patterns. A "sinus beat" pattern may be predominantly intrinsic if the cardiovascular device is sensing one or more heart chamber(s) but is not presently pacing any of them. An "AP-VS" pattern may occur when one or both atria are paced and one or both ventricles are sensed. An "AS-VP" pattern may occur when one or both atria are sensed and one or both ventricles are paced. An "AP-VP" pattern may occur when at least one atrium and at least one ventricle are paced. The model may recognize any of these patterns by their features and/or waveshape, and in some cases may identify a make or model of cardiovascular device by its "signature" SAs.

Other variations are possible; wide diversity exists between patients' intrinsic heartbeats and the customizations of cardiovascular devices grow increasingly complex as technologies advance. Moreover, a patient whose device only activates when needed may have both an intrinsic normal template (learned while the device is inactive) and a device-modulated normal template (learned while the device is active). Within any of these divisions, the data may form separated clusters based on one variable or another that may eventually become distinct templates.

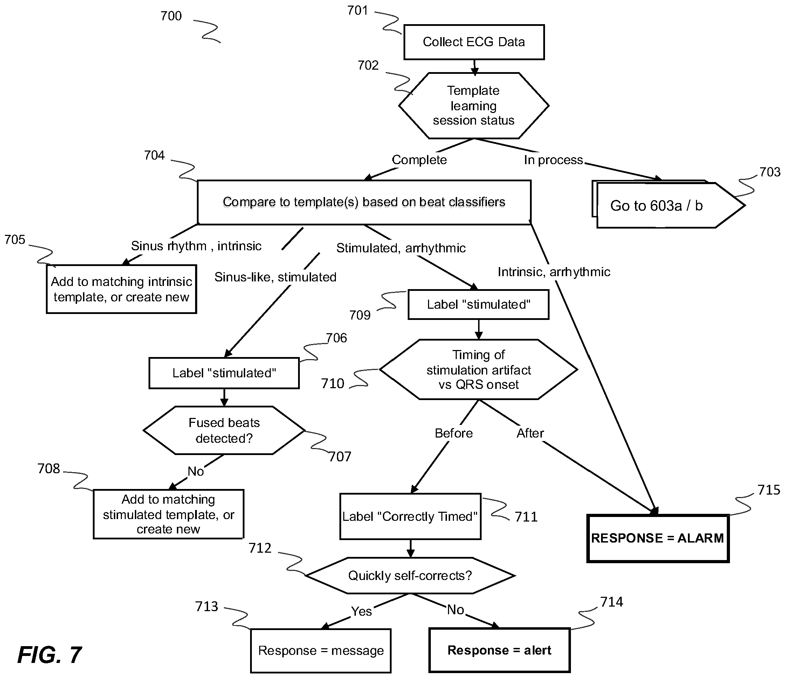

FIG. 7 is a decision tree of an ECG diagnostic analysis using knowledge-based beat classifiers according to some disclosed methods. Method 700 identifies pathologies, such as arrhythmias, revealed by ECG.

Process 701 collects ECG test data in the course of monitoring a patient's physiological parameters. Optionally, the ECG data set may be a 20-second trace or some other suitable length of time. The data set may be collected through any suitable number of leads, such as 1, 2, 5, 6, or 12.

In some embodiments, diagnosis method 700 pauses by default during a normal-template learning cycle, so branch point 702 determines whether a normal-template learning cycle is currently ongoing. If so, it takes the new test data back to process 603a/b of the template learning method 600 in FIG. 6. If the model has completed a learning cycle, it continues the diagnosis process by comparing the new data to templates based on beat classifiers in process 704. The model determines whether the new ECG data set is intrinsic or device-modulated, and whether it is normal (sinus or successfully paced) or arrhythmic (potentially harmful).

If the ECG is an intrinsic sinus beat at an acceptable rate, it is added to a matching intrinsic template or, if no matches are found in the data structure, assigned to a new one in process 705. If the ECG is "sinus-like" (acceptable rate with one or more SAs indicating successful device functioning), it is labeled, e.g., "stimulated" or "paced," in process 706. (This label should not be confused with the "flag" in template-learning process 600. A label represents an output value from the model, while a flag is an input value that describes the characteristics of such labels in datasets). If there are no fused beats detected at branch point 707, the data set may be added to an existing matching stimulated template, or assigned to a new one if not match is found, in process 708.

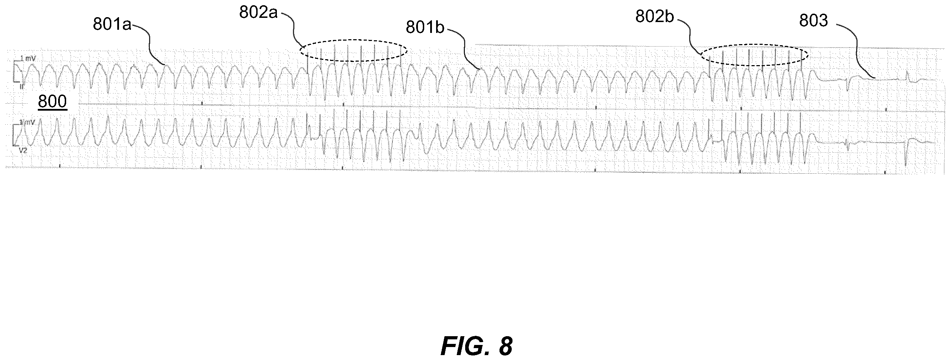

If the new ECG data set is stimulated but arrhythmic, it is labeled "stimulated" (or "paced," or any other useful descriptor) in process 709. After that, other distinctions are drawn to distinguish the specific type of arrhythmia. At branch point 710, the timing of the SA relative to the QRS complex is examined. In many cases, the SA preferably precedes the QRS onset. These ECG data sets are labeled "Correctly Timed" (or other useful descriptor) in process 711, then examined at branch point 712 for whether the arrhythmia self-corrects in a short time (e.g., less than 20 seconds) as shown in FIG. 8.

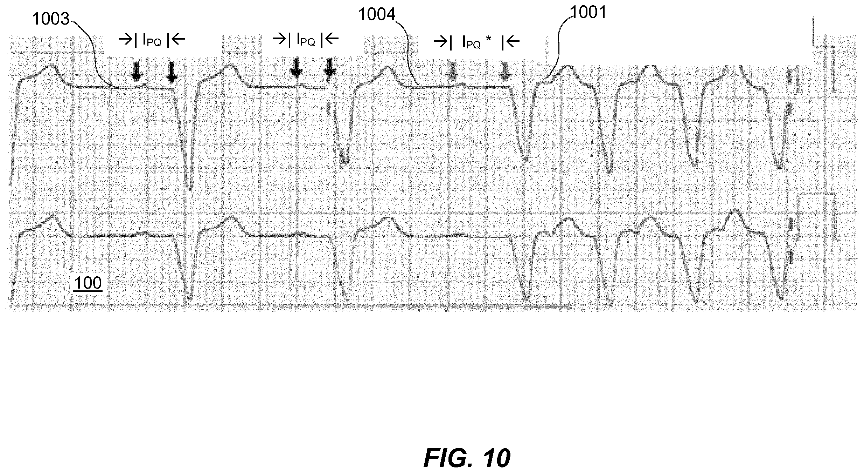

If so, the arrhythmia was corrected by the cardiovascular device and the patient does not need attention (unless it recurs more often than expected, which can also be tracked by the model). In the example, this result triggers a low-priority response such as a non-urgent message in process 713, If the arrhythmia persists, it may be an endless-loop tachycardia as shown in FIG. 10, which usually causes discomfort but not distress. This result triggers a real-time non-emergency alert in process 714 for a clinician to treat the patient's discomfort.

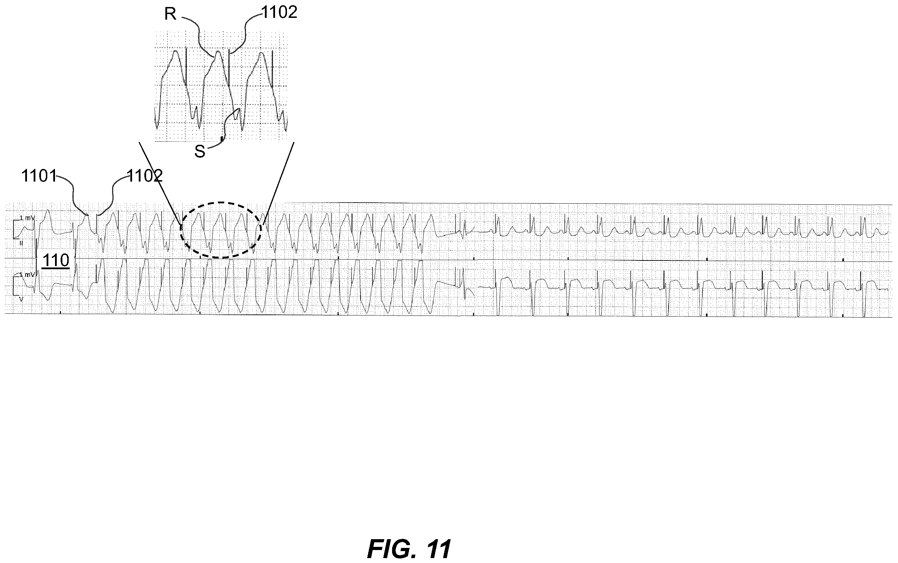

Returning to branch point 710, if the SA appears *after* the QRS onset, it may signal a more serious condition, such as the pacemaker-mediated exit-block tachycardia shown in FIG. 11. This result triggers an emergency alarm in process 715 for immediate clinician intervention.

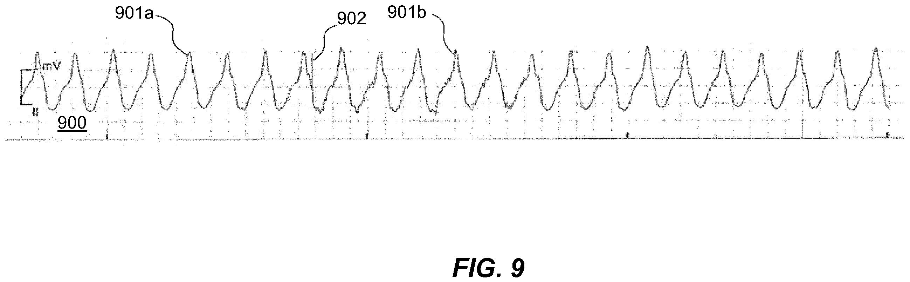

Another possible result of comparison process 704 is an intrinsic arrhythmia with no SAs in the trace. This may be an intrinsic tachycardia uncorrected by a cardiovascular device as in FIG. 9. Either the patient has no cardiovascular device, or the one they have is not functioning. Because this is also a very serious condition, such a result will also trigger an emergency alarm in process 715 for immediate clinician intervention.

The illustrated order of these processes is intended as a non-limiting example. The order of some processes may be swapped, and/or some processes may proceed in parallel, without exceeding the scope of the claims.

FIG. 8 is an example ECG trace of intrinsic tachycardia successfully corrected by a cardiovascular device. In ECG 800, intrinsic ventricular tachycardia is identifiable in the shape, rate, and amplitude of pulse train 801a. A cardiovascular device detects the tachycardia and responds with a series of pacing pulses 802a that appear superposed on the intrinsic pulse train. Afterward, the pulse train 801b still shows the signs of tachycardia. The cardiovascular device may detect the persistence of the tachycardia, or its programmed routine may be to follow its first pacing pulse series 802a with a second series 802b. Pacing pulses 802b effectively stop the tachycardia. The subsequent pulse train 803 is much closer to normal in rate, amplitude, and shape. The time allotted for a cardiovascular device to correct an intrinsic tachycardia may vary according to the manufacturer's algorithm, but typically it may comply with the Association for the Advancement of Medical Instrumentation ("AAMI") standard of 10 seconds or less.

The pacing pulses 802a, 802b corrected the tachycardia while causing the patient little or no pain. If a physiological monitoring system had immediately alerted clinicians when the tachycardia first began, the clinicians might have intervened with vigorous CPR and/or a strong shock from a paddle defibrillator. In some situations, such measures may confer a benefit that outweighs the risk of collateral harm. Overtreatment of self-terminating ventricular tachycardia is more common when the cardiovascular device immediately begins administering pacing pulses rather than, for example, waiting 6-15 seconds and delivering a stronger shock. However, if the cardiovascular device is performing effectively, such vigorous interventions may reduce the patient's quality of life and impose extra wear and tear on the cardiovascular device. Moreover, emergency responses triggered by false alarms are costly for clinics, patients, and healthcare systems.

In some embodiments, a beat classifier based in part on data resembling ECG 800 may be included in the analysis model's data structure. Because the pacemaker has corrected the tachycardia, clinician intervention is generally not warranted and the linked response may include a log entry, note to file, or low-priority message to the clinician. Optionally, the model may note number and frequency of these occurrences: Although each incident standing alone may not be cause for clinician concern, a number or frequency of incidents that exceed a predetermined threshold may warrant closer monitoring or additional tests for the patient.

FIG. 9 is an example ECG trace of intrinsic tachycardia that a cardiovascular device unsuccessfully attempts to correct. ECG 900 shows a pulse train 901A identifiable as intrinsic ventricular tachycardia. Spike 902 may be a single pacing pulse from a cardiovascular device attempting to correct the arrhythmia, or it may be something else. In any case, it is neither the rhythmic series of pacing pulses of a well-functioning pacemaker nor the higher-amplitude pulse of an ICD. Therefore, the cardiovascular device either did not detect tachycardia 901a or it tried to respond but failed for some reason. Following single pacing pulse 902 (or other spike of unknown origin), tachycardia 901a continues unabated as 901B.

This type of uncorrected tachycardia may cause severe distress and/or risk to a patient. Immediate clinician intervention may be desirable. In some embodiments, a beat classifier based in part on data resembling ECG 900 may be included in the analysis model's data structure. The linked response may include an alarm requesting emergency intervention from one or more clinicians over a communication network. Consequently, any occasion where a monitored patient's ECG data set resembles ECG 900, appropriate clinician(s) will be immediately alerted to alleviate the patient's distress.

FIG. 10 is an example ECG trace of endless-loop tachycardia ("ELT"), a type of pacemaker-mediated tachycardia. The term "pacemaker-mediated tachycardia" may be used to describe a variety of situations where a cardiovascular device paces the ventricles at inappropriately high rates. ELT affects users of dual-chamber pacemakers and similar cardiovascular devices that provide ventricular pacing inhibition. ELT is generally associated with retrograde auroventricular ("AV") duction (an electrical current in an opposite-of-normal direction through or near the AV node).

After some patients were observed to be sensitive to right-ventricle pacing, to the point of increased risk of atrial fibrillation and/or heart failure, cardiovascular device manufacturers introduced algorithms to inhibit RV pacing under certain conditions such as heart rates approaching the programmed upper rate limit of the cardiovascular device. Some of these algorithms had the unintended consequence of causing retrograde VA conduction, resulting in ELT. Symptoms of ELT may include palpitations, light headedness, syncope, or chest pain - uncomfortable, but not severe or risky like the atrial fibrillation that the inhibition algorithms were developed to prevent.

ELT may be classified as a reentrant tachycardia, meaning that an extra conductive circuit is formed in the heart that may discharge between normal beats, causing premature or extra beats that become a continuous tachycardia. The cardiovascular device may form the antegrade limb of the reentrant circuit, and the patient's intrinsic AV node (or alternatively an accessory pathway) forms the retrograde limb. Atrial-sensing, ventricular-pacing ("AS-VP") cardiovascular devices are most commonly associated with EVL, although similar behavior has been reported in atrial pacing, ventricular-pacing ("AP-VP") cardiovascular devices.

ECG data set 1000 illustrates the onset of ELT. Signal 1003 includes the action of an atrial-sensing, ventricular-pacing cardiovascular device. At this point, the heart rate is within an acceptable range. The atrial sensing coincides with the onset of the P wave (very small bump after the long pause) and the ventricular pacing pulse coincides with the onset of the QRS complex (though the Q wave is absent in this ECG, a not uncommon phenomenon). Signal 1003 has a normal PQ interval I_{PQ} of about 180ms. At 1004 there is a beat with an unusually long PQ interval I_{PQ}* of about 280ms. After that, ELT 1001 begins.

A beat classifier in the tachycardia-detection data structure may be based in part on traces resembling ECG data set 1000 to enable the model to recognize cases of ELT. The linked response may include an immediate non-emergency clinician alert to address the patient's potential discomfort. Optionally, if the model has identified the patient as pacing-dependent (because their normal templates are all, or nearly all, device-modulated), the response may be escalated from alert to alarm.

FIG. 11 is an example ECG trace of pacemaker-mediated exit-block ventricular tachycardia. This arrhythmia is unusual; only a minority of clinicians have encountered it as of this writing. On the other hand, it can place a pacing-dependent patient at serious risk. It has been managed by optimizing the programming of the cardiovascular device and, in some cases, ablating scar tissue that has formed on the myocardium. However, it must first be correctly diagnosed.

In some patients, scarring or other electrically anomalous tissue (sometimes called "refractory myocardium" because it does not conduct electricity in the same way as normal myocardium) may develop in the heart. In mild cases, the depolarizing and repolarizing currents may be able to circumvent the refractory regions, but this work-around may become less effective if it leads to formation of additional refractory tissue or ectopic circuits (abnormal current paths whose conduction is out-of-sync with the sinoatrial node and/or pacemaker).

When cardiovascular devices such as pacemakers or ICDs are implanted, some patients may develop scar tissue around the leads of the deice where they enter the myocardium. Electrolyte abnormalities or stereotactic radiation may also create or enlarge refractory regions at this critical interface. If the refractory regions block too much current from exiting the device a ventricular tachycardia known as " pacemaker exit-block-mediated ventricular tachycardia" may occur. The patient may experience multiple tachycardia episodes per day, each raising the heart rate to as much as 150 beats per minute.

Particularly in pacing-dependent patients, exit-block tachycardia may be both distressing and dangerous, warranting an emergency response. An effective response, which may include ablating the scar tissue, depends on prompt and accurate diagnosis. The disclosed machine-learning model may identify any pacing-dependent patients by the absence of fully intrinsic ECGs in their normal templates, and optionally flag them for close monitoring and escalated responses. Additionally, one or more exit-block beat classifiers may be based in part on an ECG trace such as trace 1100, with a linked response that may include an immediate emergency alarm to one or more clinicians.

As health-care improvements increase patient longevity, more patients may live longer with cardiovascular devices and some conditions that are now thought rare may become more common. Diagnosis of rare or emergent conditions such as pacemaker exit-block tachycardia is an area where some disclosed embodiments may be especially beneficial. Even when few clinicians have encountered a condition in their reading or experience, a software developer may upload a relevant beat classifier to a network as soon as it is sufficiently tested and approved. Any patient monitoring systems connected to the network can then add the new beat classifier to their machine-learning models as an update. Optionally, the response to detecting an unusual condition may include links to relevant peer-reviewed research papers so that local clinicians can quickly inform themselves on how to proceed once the patient is stabilized.

Embodiments of a disclosed method include collecting electrocardiogram ("ECG") data including at least one of an intrinsic ECG signal from a patient and a stimulation artifact from a cardiovascular device used by the patient; creating a normal template from successive sets of the ECG data by unsupervised machine learning; detecting whether a specific arrhythmia is present in the ECG data by applying at least one knowledge-based beat classifier; and executing a response corresponding to the expected effect of the specific arrhythmia on the patient.

In some embodiments, the method also includes storing the normal template and the beat classifier in a data structure, wherein the data structure links the specific arrhythmia to the corresponding response. In some embodiments, the data structure stores intrinsic normal templates separately from normal templates with stimulation artifacts.

In some embodiments, the specific arrhythmia includes tachycardia.

In some embodiments, the unsupervised machine learning continues until a threshold correlation is detected between the successive sets of the ECG data.

In some embodiments, the specific arrhythmia includes temporary intrinsic tachycardia corrected by the cardiovascular device within a predetermined time limit, and the corresponding response does not include an emergency alarm or an urgent alert requesting clinician attention.

In some embodiments, the specific arrhythmia includes pacemaker-mediated endless-loop tachycardia, and the corresponding response includes an urgent alert requesting timely clinician attention.

In some embodiments, the specific arrhythmia includes pacemaker-mediated exit-block tachycardia and the corresponding response includes an emergency alarm requesting immediate clinician intervention.

In some embodiments, the specific arrhythmia includes persistent uncorrected intrinsic tachycardia, and the corresponding response includes an emergency alarm requesting immediate clinician intervention.

In some embodiments, the stimulation artifact includes a pacing pulse, and the method includes determining whether the cardiovascular device does one of (1) atrial pacing, ventricular sensing ("AP-VS"); (2) atrial sensing, ventricular pacing before QRS onset ("AS-VP") (3) atrial pacing, ventricular pacing before QRS onset ("AP-VP"); and (4) ventricular pacing during QRS ("fused beat").

In some embodiments, the method also includes inferring information about the structure, programming, or origin of the cardiovascular device from the nature of the stimulation artifact(s) in the ECG data.

In some embodiments, the method also includes updating the normal template with newly collected ECG test data from the same patient by converting the analyzed test data to training data.

Embodiments of a disclosed physiological monitoring system include at least one electrocardiogram ("ECG") port, a processor communicatively coupled to the ECG port, and a memory communicatively coupled to the ECG port and encoded with a data structure. The data structure includes a beat classifier to detect a specific arrhythmia in ECG data, a corresponding response linked to the predefined arrythmia, and a normal template learned from ECG data. The memory is also encoded with a set of instructions that, when executed by the processor, cause the processor to collect an ECG data set, including at least one of an intrinsic ECG signal from a patient and a stimulation artifact from a cardiovascular device of the patient, through the ECG port; compare the ECG data set to the normal template; and, if the ECG data set matches the normal template, update the normal template with the ECG data set.

In some embodiments, the physiological monitoring system also includes an ECG monitoring sensor communicatively coupled to the ECG port.

In some embodiments, the cardiovascular device includes a pacemaker or an implanted cardioverter defibrillator ("ICD").

In some embodiment, the physiological monitoring system also include a communication interface for coupling to an external network.

Embodiments of a disclosed non-transitory computer-readable storage medium containing instructions that, when executed, cause a processor to collect electrocardiogram ("ECG") data including at least one of an intrinsic ECG signal from a patient and a stimulation artifact from a cardiovascular device used by the patient; create a normal template from successive sets of the ECG data by unsupervised machine learning; detect whether a specific arrhythmia is present in the ECG data by applying at least one knowledge-based beat classifier; and execute a response corresponding to the expected effect of the specific arrhythmia on the patient.

A method of analyzing an electrocardiogram (ECG) of a user of a cardiovascular device that affects the ECG is substantially set forth in the foregoing description and drawings. A physiological monitoring system for analyzing an electrocardiogram (ECG) of a user of a cardiovascular device that affects the ECG, is substantially set forth in the foregoing description and drawings. A non-transitory computer-readable storage medium containing instructions that, when executed, cause a processor to of detecting arrhythmia in an electrocardiogram (ECG) of a user of a cardiovascular device that affects the ECG is substantially set forth in the foregoing description and drawings.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted.

As used herein, the article "a" or "an" is intended to have its ordinary meaning in the patent arts, namely "one or more."

The term "about" when applied to a value generally means within the tolerance range of the equipment used to produce the value, or in some examples, means plus or minus 10%, or plus or minus 5%, or plus or minus 1 %, unless otherwise expressly specified.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner.

When an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

Expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component, or a combination thereof, but should not be construed to exclude the existence of or a possibility of the addition of one or more other characteristics, numbers, operations, constituent elements, components, or combinations.

The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

The detailed description is made with reference to the accompanying drawings and is provided to assist in a comprehensive understanding of various example embodiments of the present disclosure. Changes may be made in the function and arrangement of elements discussed without departing from the spirit and scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the spirit and scope of the present disclosure.

Various modifications to the disclosure will therefore be readily apparent to those skilled in the art, and the generic principles defined herein may be applied to other variations without departing from the spirit or scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

For the avoidance of doubt, the present disclosure includes the subject matter set out in the following numbered Clauses:
Clause 1. A method, comprising:
   collecting electrocardiogram ("ECG") data including at least one of an intrinsic ECG signal from a patient and a stimulation artifact from a cardiovascular device used by the patient;
   creating a normal template from successive sets of the ECG data by unsupervised machine learning;
   detecting whether a specific arrhythmia is present in the ECG data by applying at least one knowledge-based beat classifier; and
   executing a response corresponding to the expected effect of the specific arrhythmia on the patient.
Clause 2. The method of Clause 1, further comprising storing the normal template and the beat classifier in a data structure, wherein the data structure links the specific arrhythmia to the corresponding response.
Clause 3. The method of Clause 2, wherein the data structure stores intrinsic normal templates separately from normal templates with stimulation artifacts.
Clause 4. The method of Clause 1, wherein the specific arrhythmia comprises tachycardia.
Clause 5. The method of Clause 1, wherein the unsupervised machine learning continues until a threshold correlation is detected between the successive sets of the ECG data.
Clause 6. The method of Clause 1, wherein:
   the specific arrhythmia comprises temporary intrinsic tachycardia corrected by the cardiovascular device within a predetermined time limit; and
   the corresponding response does not include an emergency alarm or an urgent alert requesting clinician attention.
Clause 7. The method of Clause 1, wherein:
   the specific arrhythmia comprises pacemaker-mediated endless-loop tachycardia; and
   the corresponding response includes an urgent alert requesting timely clinician attention.
Clause 8. The method of Clause 1, wherein:
   the specific arrhythmia comprises pacemaker-mediated exit-block tachycardia; and
   the corresponding response includes an emergency alarm requesting immediate clinician intervention.
Clause 9. The method of Clause 1, wherein:
   the specific arrhythmia comprises persistent uncorrected intrinsic tachycardia; and
   the corresponding response includes an emergency alarm requesting immediate clinician intervention.
Clause 10. The method of Clause 1, wherein the stimulation artifact comprises a pacing pulse, and further comprising determining whether the cardiovascular device does one of:
   atrial pacing, ventricular sensing ("AP-VS");
   atrial sensing, ventricular pacing before QRS onset ("AS-VP"):
      atrial pacing, ventricular pacing before QRS onset ("AP-VP"); and
      ventricular pacing during QRS ("fused beat").
Clause 11. The method of Clause 1, further comprising inferring information about the structure, programming, or origin of the cardiovascular device from the nature of the stimulation artifact(s) in the ECG data.
Clause 12. The method of Clause 1, further comprising updating the normal template with newly collected ECG test data from the same patient by converting the analyzed test data to training data.
Clause 13. A physiological monitoring system, comprising:
   at least one electrocardiogram ("ECG") port;
   a processor communicatively coupled to the ECG port; and
   a memory communicatively coupled to the ECG port and encoded with:
      a data structure including:
         a beat classifier to detect a specific arrhythmia in ECG data;
         a corresponding response linked to the predefined arrythmia; and
         a normal template learned from ECG data; and
      a set of instructions that, when executed by the processor, cause the processor to:
         collect an ECG data set, including at least one of an intrinsic ECG signal from a patient and a stimulation artifact from a cardiovascular device of the patient, through the ECG port;
         compare the ECG data set to the normal template; and
         if the ECG data set matches the normal template, update the normal template with the ECG data set.
Clause 14. The system of Clause 13, further comprising an ECG monitoring sensor communicatively coupled to the ECG port.
Clause 15. The system of Clause 13, wherein the cardiovascular device comprises a pacemaker or an implanted cardioverter defibrillator ("ICD").
Clause 16. The system of Clause 13, further comprising a communication interface for coupling to an external network.
Clause 17. A non-transitory computer-readable storage medium containing instructions that, when executed, cause a processor to:
   collect electrocardiogram ("ECG") data including at least one of an intrinsic ECG signal from a patient and a stimulation artifact from a cardiovascular device used by the patient;
   create a normal template from successive sets of the ECG data by unsupervised machine learning;
   detect whether a specific arrhythmia is present in the ECG data by applying at least one knowledge-based beat classifier; and
   execute a response corresponding to the expected effect of the specific arrhythmia on the patient.
Clause 18. A method of analyzing an electrocardiogram (ECG) of a user of a cardiovascular device that affects the ECG, substantially as set forth in the foregoing description and drawings.
Clause 19. A physiological monitoring system for analyzing an electrocardiogram (ECG) of a user of a cardiovascular device that affects the ECG, substantially as set forth in the foregoing description and drawings.
Clause 20. A non-transitory computer-readable storage medium containing instructions that, when executed, cause a processor to of detecting arrhythmia in an electrocardiogram (ECG) of a user of a cardiovascular device that affects the ECG, substantially as set forth in the foregoing description and drawings.

## Claims

1. A method, comprising:
collecting electrocardiogram ("ECG") data including at least one of an intrinsic ECG signal from a patient and a stimulation artifact from a cardiovascular device used by the patient;
creating a normal template from successive sets of the ECG data by unsupervised machine learning;
detecting whether a specific arrhythmia is present in the ECG data by applying at least one knowledge-based beat classifier; and
executing a response corresponding to the expected effect of the specific arrhythmia on the patient.

2. The method of Claim 1, further comprising storing the normal template and the beat classifier in a data structure, wherein the data structure links the specific arrhythmia to the corresponding response, optionally wherein the data structure stores intrinsic normal templates separately from normal templates with stimulation artifacts.

3. The method of any one of Claims 1 or 2, wherein the specific arrhythmia comprises tachycardia.

4. The method of any one of Claims 1 to 3, wherein the unsupervised machine learning continues until a threshold correlation is detected between the successive sets of the ECG data.

5. The method of any one of Claims 1 to 4, wherein:
the specific arrhythmia comprises temporary intrinsic tachycardia corrected by the cardiovascular device within a predetermined time limit; and
the corresponding response does not include an emergency alarm or an urgent alert requesting clinician attention.

6. The method of any one of Claims 1 to 5, wherein:
the specific arrhythmia comprises pacemaker-mediated endless-loop tachycardia; and
the corresponding response includes an urgent alert requesting timely clinician attention.

7. The method of any one of Claims 1 to 6, wherein:
the specific arrhythmia comprises pacemaker-mediated exit-block tachycardia; and
the corresponding response includes an emergency alarm requesting immediate clinician intervention.

8. The method of any one of Claims 1 to 7, wherein:
the specific arrhythmia comprises persistent uncorrected intrinsic tachycardia; and
the corresponding response includes an emergency alarm requesting immediate clinician intervention.

9. The method of any one of Claims 1 to 8, wherein the stimulation artifact comprises a pacing pulse, and further comprising determining whether the cardiovascular device does one of:
atrial pacing, ventricular sensing ("AP-VS");
atrial sensing, ventricular pacing before QRS onset ("AS-VP"):
atrial pacing, ventricular pacing before QRS onset ("AP-VP"); and
ventricular pacing during QRS ("fused beat").

10. The method of any one of Claims 1 to 9, further comprising:
a) inferring information about the structure, programming, or origin of the cardiovascular device from the nature of the stimulation artifact(s) in the ECG data; and /or
b) updating the normal template with newly collected ECG test data from the same patient by converting the analyzed test data to training data.

11. A physiological monitoring system, comprising:
at least one electrocardiogram ("ECG") port;
a processor communicatively coupled to the ECG port; and
a memory communicatively coupled to the ECG port and encoded with:
a data structure including:
a beat classifier to detect a specific arrhythmia in ECG data;
a corresponding response linked to the predefined arrythmia; and
a normal template learned from ECG data; and
a set of instructions that, when executed by the processor, cause the processor to:
collect an ECG data set, including at least one of an intrinsic ECG signal from a patient and a stimulation artifact from a cardiovascular device of the patient, through the ECG port;
compare the ECG data set to the normal template; and
if the ECG data set matches the normal template, update the normal template with the ECG data set.

12. The system of Claim 11, further comprising an ECG monitoring sensor communicatively coupled to the ECG port.

13. The system of any one of Claims 11 or 12, wherein the cardiovascular device comprises a pacemaker or an implanted cardioverter defibrillator ("ICD").

14. The system of any one of Claims 11 to 13, further comprising a communication interface for coupling to an external network.

15. A non-transitory computer-readable storage medium containing instructions that, when executed, cause a processor to:
collect electrocardiogram ("ECG") data including at least one of an intrinsic ECG signal from a patient and a stimulation artifact from a cardiovascular device used by the patient;
create a normal template from successive sets of the ECG data by unsupervised machine learning;
detect whether a specific arrhythmia is present in the ECG data by applying at least one knowledge-based beat classifier; and
execute a response corresponding to the expected effect of the specific arrhythmia on the patient.
